# EUROPÄISCHE PATENTSCHRIFT

(11) **EP 2 626 424 B1**
(45) Veröffentlichungstag und Bekanntmachung des Hinweises auf die Patenterteilung: **02.09.2015**
(21) Anmeldenummer: 12000788.5
(22) Anmeldetag: 07.02.2012
(51) Int. Cl.: C12N 11/14, C12N 11/04

(54) **Verfahren zum Konservieren von lebenden oder lebensfähigen Mikroorganismen und Mikroorganismen-Substanz**
Method for preserving living or life-capable micro-organisms and micro-organism substance
Procédé de conservation de micro-organismes et substances de micro-organismes vivants ou capables de vivre

(43) Veröffentlichungstag der Anmeldung: 14.08.2013
(73) Patentinhaber: Schneider-Planta Chemicals GmbH, 66571 Eppelborn-Dirmingen (DE)
(72) Erfinder: Jahns, Thomas, Dr., 66119 Saarbrücken (DE); Schneider, Detlef, 66125 Saarbrücken (DE); Planta, Ulrike, 66636 Tholey (DE)
(74) Vertreter: Wunderlich, Rainer

(56) Entgegenhaltungen:
- JP-A- 11 137 244
- US-A1- 2003 165 472
- HAIJIE LIU ET AL: "CHALK CULTURE METHOD FOR PRESERVING BACTERIA CULTURES AND ITS APPLICATIONS", JOURNAL OF RAPID METHODS AND AUTOMATION IN MICROBIOLOGY, Bd. 11, Nr. 3, 1. November 2003 (2003-11-01), Seiten 163-224, XP55031593, ISSN: 1060-3999, DOI: 10.1111/j.1745-4581.2003.tb00041.x

## Beschreibung

Die Erfindung betrifft ein Verfahren zum Konservieren von lebenden oder lebensfähigen Mikroorganismen gemäß Patentanspruch 1 sowie eine Mikroorganismen-Substanz gemäß Patentanspruch 10.

Mikroorganismen, wie Hefepilze oder Bakterien, werden heutzutage in der Landwirtschaft, der Futtermittelherstellung, der Pharmaindustrie, beim Ölabbau, der Entsorgungs- und Dekontaminationstechnik und vielen anderen Bereichen verwendet. In vielen Fällen ist es gewünscht oder erforderlich, große Mengen der Mikroorganismen zu Lagern, um sie einer späteren Verwendung bedarfsgerecht zuzuführen. Bei den hierzu vorgesehenen Präparaten besteht das Problem, dass Mikroorganismen in einer normalen Umwelt häufig bereits nach wenigen Stunden absterben.

Aufgrund der Empfindlichkeit und/oder kurzen Lebensdauer der Mikroorganismen müssen diese zur Lagerung in besonderer Weise konserviert werden. Die am häufigsten verwendeten Konservierungstechniken sind das Einfrieren beispielsweise in Glycerin sowie das Gefriertrocknen. Mit diesen beiden Verfahren werden zwar zufriedenstellende Überlebensraten erzielt, aber diese Verfahren sind sehr kostenaufwändig. Zudem sind die Mikroorganismen hierbei starken Belastungen ausgesetzt.

Bekannt ist weiter, Mikroorganismen in evakuierten oder mit speziellem Schutzgas befüllten Glasbehältem zu lagern. Dies ist aber nur für kleinere Mengen möglich und ebenfalls mit relativ hohen Kosten verbunden.

Ausgehend von diesem Stand der Technik liegt der Erfindung daher die **Aufgabe** zugrunde, ein Verfahren zum Konservieren von lebenden oder lebensfähigen Mikroorganismen anzugeben, bei welchem die Mikroorganismen in besonders effizienter Weise und gleichwohl schonend konserviert werden können. Ferner liegt der Erfindung die Aufgabe zugrunde, eine entsprechende Mikroorganismen-Substanz zur Verfügung zu stellen.

Diese Aufgabe wird zum einen durch ein Verfahren gemäß Anspruch 1 gelöst. Dabei bilden Mikroorganismen mit einem flüssigen Kulturmedium eine Mikroorganismensuspension, mit welchem pulverförmiges poröses Substrat beimpft wird, wobei sich Mikroorganismen in und an Partikeln des porösen Substrates anlagern und anschließend die mit den Mikroorganismen versehenen Partikel des Substrates mit einem Hüllmaterial luftdicht umschlossen und zu Formkörpern geformt werden.

Zum anderen wird die Aufgabe durch eine Mikroorganismen-Substanz gemäß den Merkmalen des Anspruchs 10 gelöst. Bei dieser Konservierungssubstanz, welche mit einem pulverförmigen porösen Substrat gebildet ist, deren Partikel mit lebenden oder lebensfähigen Mikroorganismen beimpft sind, sind zum Konservieren der Mikroorganismen die mit Mikroorganismen beimpften Partikel des Substrates mit einem Hüllmaterial luftdicht umschlossen und zu Formkörpern geformt. Vorteilhafte Ausgestaltungen sind in den jeweils abhängigen Ansprüchen ausgeführt.

Durch die erfindungsgemäßen Merkmale wird ein Verfahren zum Konservieren von lebenden oder lebensfähigen Mikroorganismen bereitgestellt, bei welchem auch größere Mengen an Mikroorganismen effizient konserviert und in einem lebensfähigen Zustand erhalten werden können.

Ein erster Aspekt der Erfindung liegt darin, die Mikroorganismen in oder an einem pulverförmigen porösen Substrat anzulagern. Dieses poröse Substrat bietet eine große Oberfläche, so dass eine gute Besiedelung durch die Mikroorganismen erreicht wird. Das mitaufgebrachte flüssige Kulturmedium kann diese Besiedelung fördern.

Gemäß einem zweiten Aspekt werden die besiedelten Partikel des Substrates luftdicht abgekapselt.

Durch das luftdichte Hüllmaterial wird der Sauerstoffzutritt zu den sauerstoffempfindlichen Mikroorganismen verhindert und dadurch deren Überlebensrate erheblich gesteigert.

Eine stabile Abkapselung wird dadurch erreicht, dass die umschlossenen Pulverpartikel zu größeren Formkörpern, etwa von 1 mm bis einigen Zentimetern, geformt werden. Hierdurch sind diese leicht zu handhaben und für große Anwendungen und Massenanwendungen, etwa in der Landwirtschaft, der Dekontamination großer Flächen oder im industriellen Maßstab setzbar. Bei dem erfindungsgemäß hergestellten Präparat werden auch nach vielen Wochen und Monaten hohe Überlebensraten von ca. 90% erreicht.

Als zu konservierende Mikroorganismen kommen insbesondere Bakterien, Pilze, Sporen, Viren, Algen etc. in Frage. Das Beimpfen des pulverförmigen porösen Substrates mit der Mikroorganismensuspension kann dabei beispielsweise durch Vermischen oder Aufsprühen erfolgen.

Die Einwirkzeit der Mikroorganismensuspension auf dem porösen Substrat kann sich dabei in einem Bereich von einigen Minuten bis zu mehreren Stunden bewegen. Um die Überlebensfähigkeit der Mikroorganismen noch weiter zu erhöhen, können diese vorab einer sogenannten "Stressbehandlung" unterzogen werden. Hierbei wird etwa der osmotische Druck des Kulturmediums, zum Beispiel der Nährflüssigkeit, durch Hinzufügen von Salz, Zucker oder anderer osmotisch aktiver Substanzen erhöht. Dadurch werden die Mikroorganismen dazu angeregt, bestimmte eigene Substanzen zu erzeugen, um dem erhöhten osmotischen Druck entgegenzuwirken. Hierdurch wird eine Erhöhung der Widerstandsfähigkeit der Mikroorganismen bewirkt.

Als poröses Substrat können grundsätzlich verschiedene natürliche oder synthetische poröse Materialien wie Sandstein, Kalkstein, Dolomit, Tonstein sowie Schieferton oder geeignete Kunststoffe wie Polyethylen, Polypropylen, Polyvinylchlorid, Polystyrol, Polyurether oder Polyethylenterephthalat verwendet werden, die beispielsweise durch Gas erzeugende Zusatzstoffe aufgeschäumt sind. Auch poröse Naturstoffe wie Cellulose können eingesetzt werden.

Nach der Erfindung ist es vorteilhaft, wenn das pulverförmige poröse Substrat Kieselgur umfasst.

Kieselgur, auch Diatomeenerde genannt, weist eine große äußere und innere Oberfläche auf. Obwohl Kieselgur als ein natürliches Biozid gilt, hat sich hierbei eine sehr hohe Überlebensrate auch bei längerer Lagerzeit ergeben. Es ist davon auszugehen, dass Kieselgur eine gewisse Schutzwirkung für die angesiedelten Mikroorganismen bewirkt.

Es ist nach der Erfindung bevorzugt, dass als Hüllmaterial ein thermoplastisches wasserlösliches Polymer, insbesondere Polyethylenglykol (PEG), eingesetzt wird.

Thermoplaste lassen sich insbesondere in einem Extruder in einem bestimmten Temperaturbereich erweichen oder verflüssigen. In diesem Zustand ist eine gute Umhüllung der Partikel möglich. Dabei ist dieser Vorgang reversibel, so dass durch Abkühlung eine stabile Hülle erreicht werden kann. Die Wasserlöslichkeit ist zweckmäßig, damit die gebildeten und verfestigten Formkörper nach dem Ausbringen durch das Auflösen des Hüllmaterials die Mikroorganismen in einem definierten Zeitraum freisetzen können. Die Wasserlöslichkeit der thermoplastischen Polymeren kann insbesondere durch die Auswahl einer geeigneten Molmasse gezielt gesteuert werden. Bei Verwendung von Polyethylenglykol sind insbesondere Molekulargewichte von 4000, 8000 und 20000 g/mol geeignet, wobei die Wasserlöslichkeit mit steigendem Molekulargewicht abfällt. Es kann so eine definierte Freisetzung der Mikroorganismen gesteuert werden.

Als Thermoplaste können neben Polyethylenglykol etwa auch Acrylnitryl, Butadenstyrol, Polyamide, Polylactate, Polymethylmethacrylate, Polycarbonate, Polyethylenterephthalate, Polyethylen, Polypropylen, Polystyrol, Polyether, Polyethanketon sowie Polyvinylchlorid vorgesehen werden. Dabei sollte der Schmelzpunkt der verwendeten Thermoplaste nicht oder nicht erheblich über 55°C liegen, da oberhalb dieser Temperatur die Mikroorganismen absterben können. Das Hüllmaterial kann auch in mehreren Schichten wechselweise zu den Partikeln des porösen Substrates aufgetragen sein, um eine definierte steuerbare Zerfallskinetik mit unterschiedlichen Freisetzungsintervallen der Mikroorganismen zu ermöglichen. Auf diese Weise können die Mikroorganismen über einen sehr langen Zeitraum kontrolliert an die Umgebung abgegeben werden.

Ein wesentlicher Vorteil von Polyethylenglykol ist neben seiner Wasserlöslichkeit die Hygroskopizität sowie die niedrige Flüchtigkeit und die hohe thermische Stabilität. Darüber hinaus weisen Polyethylenglykole außergewöhnliche Toxizitätswerte auf. Bei niedrigen Molmassen ist Polyethylenglykol ferner auch biologisch abbaubar.

Es ist zweckmäßig, wenn das flüssige Kulturmedium ein Komplexmedium oder ein definiertes synthetisches Medium ist.

Mit diesen Grundsubstanzen ist die Nährstoffversorgung der Mikroorganismen während des Zeitraums der Besiedelung und/oder Lagerung gewährleistet. Als Energiequelle können neben organischen Stoffen auch Schwefelverbindungen eingesetzt werden. Benötigte Nährstoffe beinhalten insbesondere organische oder anorganische Kohlenstoff-, Stickstoff-, Schwefel- und Phosphatquellen sowie andere essenzielle Nährstoffe. Neben Zucker können Proteinhydrolysate und Fettsäuren als Nährstoffe Verwendung finden. Zusätzlich können anorganische Salze dem Mikroorganismus lebenswichtige Ionen und Moleküle, wie zum Beispiel Ammonium, Kalium, Natrium, Phosphat, Sulfat sowie Spurenelemente liefern. Daneben können noch enthalten sein Puffersubstanzen um den pH-Wert zu stabilisieren, Indikatoren um Änderungen anzuzeigen, wie zum Beispiel beim pH-Wert, oder auch um gewisse Stoffwechselprodukte oder Stoffwechselaktivitäten anzuzeigen, Farbstoffe beziehungsweise deren Vorstufen, Hemmstoffe zum Beispiel Antibiotika und selektive Agenzien, um das Wachstum unerwünschter Mikroorganismen zu verhindern zum Beispiel Chloramphenicol, Wachstumshilfsstoffe wie Hormone, Vitamine und dergleichen sowie Gelierungsmittel, wie Agar-Agar oder Gelatine. Das verwendete Wasser ist vorzugsweise destilliert oder demineralisiert.

Bei dem erfindungsgemäßen Verfahren wird das Verfahren unter Verwendung eines Extruders durchgeführt, wobei zunächst das Hüllmaterial und anschließend das mit den Mikroorganismen beimpfte poröse Substrat in den Extruder eingeleitet und darin zu einer Formmasse vermischt wird, welche zu den Formkörpern ausextrudiert wird.

Mit einem Extruder kann die Vermischung von Hüllmaterial und das mit den Mikroorganismen beimpfte poröse Substrat schnell und mit dem gewünschten Homogenitätsgrad unter wirtschaftlich günstigen Bedingungen in einer einzelnen Vorrichtung erreicht werden. Die Extrudertechnik ist eine bewährte und ausgereifte Technologie, die nur wenig Bedienpersonal und einfache Wartung erfordert. Dabei wird aus den einzelnen Komponenten in dem Extruder unter Verwendung der Feuchtigkeit des Kulturmediums durch die mechanischen Belastungen die plastische Masse gebildet. Der Extruder kann ein 1-Wellen- oder Mehrwellenextruder sein, welcher etwa gleichoder gegenläufig antreibbar ist. Bei einem Mehrwellenextruder sind zwei kämmende, gleichlaufende Extruderschnecken bevorzugt, wobei das Verfahren besonders schnell und schonend durchgeführt werden kann. Durch ein zwei- oder mehrstufiges Zugeben der einzelnen Komponenten, zunächst das Hüllmaterial und später das mit den Mikroorganismen beimpfte poröse Substrat, können hierfür jeweils verschiedene Druck- und Temperaturbedingungen eingestellt werden.

Geeigneterweise wird zum Bilden der Formmasse eine Trägersubstanz in den Extruder eingeleitet.

Die Trägersubstanz mildert die während der Extrusion auf die Mikroorganismen wirkenden Scherkräfte ab und erhöht dadurch deren Überlebensrate. Ferner kann durch die Trägersubstanz die Dichte der erhaltenen Formkörper definiert auf den jeweiligen Anwendungszweck eingestellt werden. Insbesondere die mechanische Stabilität der Formkörper ist auf diese Weise gezielt variierbar. Erfindungsgemäß beträgt der Anteil der Trägersubstanz zwischen 60% und 90%, insbesondere etwa 80% der Masse des Formkörpers. Das beimpfte Substrat hat einen Anteil von ca. 2%, während der Restbestandteil durch das Hüllmaterial gebildet ist.

Geeigneterweise wird die Trägersubstanz zusammen mit dem Hüllmaterial in den Extruder eingeleitet.

Dadurch kann der gleiche apparative Einlauf für die Trägersubstanz und das Hüllmaterial verwendet werden, und es entfallen zusätzliche Einrichtungen. Ferner wird auf diese Weise die Trägersubstanz unmittelbar mit dem Hüllmaterial in dem Extruder vermischt, so dass bereits eine homogene Vermischung der Trägersubstanz mit dem Hüllmaterial auf das mit den Mikroorganismen beimpfte poröse Substrat in den Extruder trifft. Der energetische Aufwand und die Effizienz des Verfahrens werden somit verbessert.

Für eine besonders gute Dämpfungswirkung umfasst die Trägersubstanz Bentonit, Rapsschrot, Dicalciumphosphat und/oder Kreide.

Bentonit, als natürliches Tonmineralgemisch, weist eine starke Quell- und Wasseraufnahmefähigkeit sowie eine gute Adsorptionsfähigkeit auf. Rapsschrot ist ein aus der Rapssaat als Koppelprodukt gewonnenes Nebenprodukt des Rapsöls und weist einen hohen Proteinanteil auf. Als nachwachsender biologischer Rohstoff ist er in großen Ressourcen vorhanden und zu niedrigen Kosten zu erhalten. Darüber hinaus ist er durch die Mikrobiologie der Umgebung vollständig abbaubar. Der Restölanteil des Rapsschrotes kann dazu dienen, die Hydrophobie des Formkörpers sowie die Fließfähigkeit der Formmasse gezielt zu beeinflussen. Es können aber auch andere biologische Stoffe eingesetzt werden, etwa Sägemehl, Strohhäcksel etc. Das Dicalciumphosphat weist hygroskopische Eigenschaften auf und bindet Wasser. Ferner dient es als Säureregulator um den pH-Wert der Formmasse zu regulieren. Die Kreide beeinflusst ebenfalls die Konsistenz der Formmasse, dient als Bindemittel und zur Abmilderung des pH-Wertes.

Es ist vorteilhaft, wenn das Hüllmaterial in einem festen Zustand in den Extruder eingeleitet, darin aufgeschmolzen und bei einer Temperatur von nicht mehr als 55°C mit dem mit den Mikroorganismen beimpften Substrat vermischt wird.

Das Hüllmaterial kann somit in einer leicht transportablen festen Form transportiert und dem Extruder zugeführt werden. Die zum Aufschmelzen des Hüllmaterials erforderliche Temperatur kann auch deutlich über 55°C liegen, etwa bei 65°C, vor dem Vermischen mit den Mikroorganismen beimpften Substrat sollte diese jedoch auf maximal 55°C beschränkt werden, um die Mikroorganismenkultur nicht zu schädigen.

Die beiden Verfahrensstufen des Aufschmelzens des Hüllmaterials sowie des Vermischens des Hüllmaterials mit dem mit den Mikroorganismen beimpften Substrat lassen sich innerhalb des Extruders geeignet in zwei getrennten räumlichen Bereichen durchführen. Für diese beiden getrennten Bereiche können jeweils Druck und Temperatur definiert eingestellt werden. Bei eventuell auftretenden Problemen innerhalb der betreffenden Verfahrensstufen lassen sich auftretende Störungen dadurch leicht erkennen und beheben. Auch die jeweiligen Zufuhrraten und -mengen können jeweils geeignet variiert werden.

Es ist vorteilhaft, wenn die Formmasse am Austritt aus dem Extruder durch eine Lochmatrize zu Strängen geformt wird, welche zu Granulat abgelängt werden.

Dadurch können die Formkörper in praktisch jeder beliebigen Geometrie und Größe hergestellt werden. Da sich über die Granulatgröße und -geometrie die spezifische Oberfläche des Granulates bestimmt, ist es möglich, über diese Parameter die Zersetzungsgeschwindigkeit der Formmasse in der Umgebung an die erforderlichen Bedingungen anzupassen. Ferner wird durch das Extrudierverfahren die notwendige mechanische Druckkraft bereitgestellt, welche für ein Hindurchpressen durch ein Formwerkzeug notwendig ist, so dass eine sehr schnelle und gleichmäßige Formgebung erreicht wird. Bei Abkühlung der extrudierten Materialstränge, welche vorzugsweise unmittelbar am Austritt aus dem Extruder mittels Schneideinrichtung abgelängt werden, härtet die Masse zu einer stabilen, festen Struktur aus.

Gegenstand der Erfindung ist auch eine Mikroorganismen-Substanz, welche mit einem pulverförmigen porösen Substrat gebildet ist, deren Partikel mit lebenden oder lebensfähigen Mikroorganismen beimpft sind, wobei zum Konservieren der Mikroorganismen die mit Mikroorganismen beimpften Partikel des Substrates mit einem Hüllmaterial luftdicht umschlossen und zu Formkörpern geformt sind. Die Substanz ist vorzugsweise mit dem vorbeschriebenen Verfahren hergestellt.

Mit einer derartigen Substanz kann eine erhebliche Anzahl von Mikroorganismen auf kleinstem Raum gespeichert und konserviert und über einen sehr langen Zeitraum in einem lebensfähigen Zustand erhalten werden, da etwa der nicht nur für die anaeroben sondern auch für die aeroben Mikroorganismen unter diesen Bedingungen toxische Sauerstoff wegen der luftdichten Umhüllung nicht in das poröse Substrat eindringen kann. Die Ausbildung zu Formkörpern erlaubt eine einfache und praktikable Ausbringung der Mikroorganismen-Substanz in die zu behandelnde Umgebung, das heißt beispielsweise auf landwirtschaftlichen Nutzflächen oder in Gewässer zum Abbau von organischen Verunreinigungen. Die Formkörper können auf diese Weise in üblichen Behältern zur längeren Lagerung aufgewahrt werden. In der inneren Oberfläche des pulverförmigen porösen Substrates können die Mikroorganismen durch spezielle Verfahren nicht nur eingebracht, sondern an der Oberfläche auch angebunden werden, um eine Immobilisierung der Mikroorganismen zu gewährleisten. Die Aufbringung der Mikroorganismen kann in Form einer Nährlösung durch Mischen, Aufsprühen oder Aufgießen auf das poröse Substrat erfolgen.

Erfindungsgemäß umfasst das poröse Substrat Kieselgur und das Hüllmaterial ein thermoplastisches wasserlösliches Polymer.

Kieselgur weist eine sehr hohe innere spezifische Oberfläche auf und kann daher eine große Menge der Mikroorganismen enthaltenden Suspension aufnehmen. Aufgrund des geringen spezifischen Gewichtes von Kieselgur ist der Transport einer großen Anzahl von Formkörpern sehr praktikabel. Durch die Thermoplastizität des Hüllmaterials kann dieses mit der Trägersubstanz in einem Extruder unter definiertem Druck und Temperatur effektiv vermischt werden. Neben einer guten Vermengung der eingesetzten Komponenten zu einer plastischen homogenen Masse wird nach einer Abkühlphase eine formstabile feste Konsistenz erhalten, die eine Formgebung durch ein Formwerkzeug am Austritt des Extruders ermöglicht.

Über die Wasserlöslichkeit des Polymeren kann die Freisetzungsgeschwindigkeit der Mikroorganismen aus dem porösen Substrat auf die Umgebung definiert auf den jeweiligen Anwendungszweck eingestellt werden. Die Wasserlöslichkeit des Polymeren hängt dabei insbesondere stark von der Molmasse des Polymeren ab sowie dem Vorliegen von hydrophilen funktionellen Gruppen. Erfindungsgemäß ist für die Konservierungssubstanz weiter eine Trägersubstanz mit einem Massenanteil von zumindest 70% vorgesehen, wobei bevorzugt die Trägersubstanz Bentonit, Rapsschrot, Dicalciumphosphat und/oder Kreide umfasst.

Bei diesem Massenanteil der Trägersubstanz werden hohe Scherkräfte auf die Mikroorganismen vermieden, welche ansonsten zu einer Zerstörung der Organismen führen würde. Darüber hinaus ist bei diesem Anteil eine ausreichend hohe Fließfähigkeit der Formmasse im Extruder gewährleistet. Ferner können weitere Additive, wie zum Beispiel Aktivkohle zugesetzt werden, welche toxische Stoffe binden.

Es ist zweckmäßig, wenn die Formkörper als Granulatkörner mit einer Granulatgröße zwischen 1 mm und 15 mm gebildet sind.

Diese Granulatgröße lässt sich mit herkömmlichen Lochmatrizen einfach, kostengünstig und in großen Mengen herstellen. Ferner kann diese Formkörpergröße ohne weitere Hilfsmittel auch von Einzelpersonen einfach ausgebracht werden. Darüber hinaus weist diese Größe ein günstiges Verhältnis von Oberfläche zu Volumen auf, um eine rasche Auflösungskinetik des wasserlöslichen Hüllmaterials zu ermöglichen. Auch können derartige Formkörper Platz sparend in handelsüblichen geeigneten Behältnissen ohne Ausbildung eines zu hohen Totvolumens aufbewahrt werden.

Die Erfindung wird nachfolgend anhand eines bevorzugten Ausführungsbeispiels näher erläutert.

Im beschriebenen Ausführungsbeispiel werden Bakterien der Art *Pseudomonas fluorescenz* verwendet, wobei auf diese Art auch andere Gram-negative und Grampositive vegetative Mikroorganismenzellen sowie Sporen von Bakterien und Pilzen verwendet werden können.

Die Mikroorganismen werden in einem Nährmedium zu gewünschten, das heißt zu exponentiellen oder stationären, Wachstumsphasen kultiviert. Als geeignete Medien können sowohl Komplexmedien, wie Nährboullion, Trypton, Peptonwasser oder ähnliches oder definierte synthetische Mineralmedien verwendet werden. Nach Erreichen der gewünschten, exponentiellen oder stationären Wachstumsphase können die Zellen direkt für die weiteren Verfahrensschritte verwendet werden. Alternativ können die Zellen kurz, das heißt für 1 bis 3 Stunden, vor der Weiterverarbeitung einem osmotischen Stress ausgesetzt werden durch Zugabe von 0,1 bis 0,5 M einer osmotisch wirksamen Substanz, zum Beispiel Natriumchlorid, welche in einigen Fällen die intrazelluläre Bildung von Osmolyten bewirkt.

Die Zellen können anschließend entweder direkt weiterverarbeitet werden, oder nach Zentrifugation, bei 10.000 g, 20 Minuten, bei Raumtemperatur, aufkonzentriert und auf gewünschte Zelldichten zwischen 10¹⁰ Zellen/ml und 10¹² Zellen/ml in einem gewünschten Suspensionsmedium eingestellt werden.

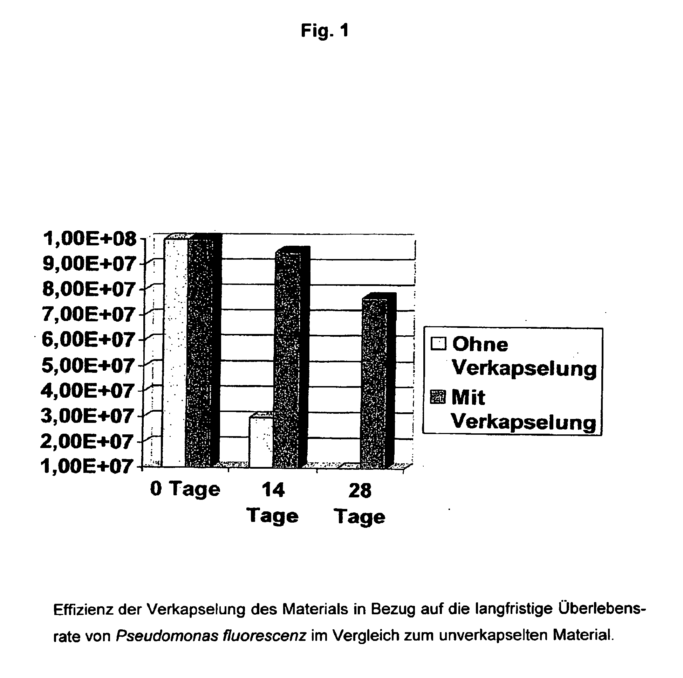

Die so erhaltene Zellsuspension wird in einem Verhältnis von einem Teil Zellsuspension mit einem Teil Kieselgur (Diatomeenerde) für zwischen 15 und 120 Minuten gründlich vermischt, wobei ein verhältnismäßig trocken erscheinendes Pulver erhalten wird. In diesem Schritt erfolgt eine Anheftung der Mikroorganismen an die innere und äußere Oberfläche des Kieselgurs. Anschließend erfolgt ein Vermischen dieses Pulvers mit einem weiteren Teil Magermilchpulver, einem weiteren Teil Saccharose sowie einem halben bis einem weiteren Teil Aktivkohle in beliebiger Reihenfolge. Die so vorbereiteten Mikroorganismen und im Folgenden als Mischgut bezeichnet werden innerhalb von 24 Stunden der Verkapselung durch eines der im Nachfolgenden genannten bevorzugten Verfahren zugeführt.
1. Verkapselung im Doppel-Z-Kneter: Vor Zugabe des Mischgutes werden 9 Teile einer Trägermasse, bestehend aus Bentonit, DCP, Polyethylenglykol 4000, Rapsschrot und Mikrosöhl (Kreide) im Doppel-Z-Kneter von zwei robusten Z-Knetarmen mit Rechts- oder Linkslauf zur Trogmitte (Trogsattel) gefördert, dort geteilt, um eine gleichmäßige Vermischung zu erhalten, und für 5 Minuten bei einer Temperatur von 50°C vorgeknetet und teilweise plastifiziert. Anschließend wird ein Teil des Mikroorganismen enthaltenden Mischgutes zugegeben, bei derselben Temperatur für 5 Minuten im Kneter weiterbehandelt, weiter plastifiziert und verkapselt, dem Doppel-Z-Kneter anschließend entnommen und auf Raumtemperatur abgekühlt.
   Die beigefügte Figur 1 zeigt die Effizienz der Verkapselung in Bezug auf die langfristige Überlebensrate der Mikroorganismen im Vergleich zum unverkapselten Material, das heißt eine Mischung des Mischgutes und der Trägermasse ohne Plastifizierung. In diesem konkreten Beispiel wurden Zellen von *Pseudomonas fluorescenz* verwendet, welche in Nährboullion bis in die stationäre Wachstumsphase angezogen wurden. Das Mischgut enthielt 10⁹ Zellen/g, die Zellzahl in der Mischung aus der Trägermasse und dem Mischgut 10⁸ Zellen/g.
2. Verkapselung im Aufbereitungsextruder: Extruder arbeiten nach dem Prinzip des Schneckenförderers, welcher feste bis dickflüssige Massen unter erhöhtem Druck und erhöhter Temperatur gleichmäßig aus einer Form gebenden Öffnung herauspresst. Der Aufbereitungsextruder dient der chemischen und/oder physikalischen Modifizierung, das heißt Reagieren, Mischen, Entgasen, Plastifizieren. Der für die Verkapselung verwendete gleichlaufende Doppelwellenextruder verfügt über zwei Schneckenwellen, welche in gleicher Drehrichtung rotieren.
   Zur Verkapselung des Mikroorganismen enthaltenden Mischgutes werden 9 Teile einer Trägermasse, bestehend aus Bentonit, DCP, Polyethylenglykol 4000, Rapsschrot und Mikrosöhl über einen Trichter gravimetrisch den Schnecken in einer sogenannten Einzugszone zugeführt und in einer ersten Teilstrecke des Extruders bei einer Temperatur von 65°C gemischt, entgast, verdichtet und plastifiziert. Als weitere Komponente wird das Mikroorganismen enthaltende Mischgut über eine in einer zweiten Extruderteilstrecke liegenden Beschickung in den Schneckenzylinder eingebracht. Diese zweite Teilstrecke wird auf einer Temperatur von 45 bis 50°C gehalten. Auf dieser zweiten Teilstrecke erfolgt ein intensives Vermischen des Mischgutes mit der Trägermasse, ein Einschließen und eine Verkapselung des Mischgutes von der Trägermasse sowie die Förderung des homogenen Gemisches zur Austragszone, am Ende derer eine Form gebende Öffnung, zum Beispiel eine Lochplatte, dem Produkt seine Form verleiht. Nach Austritt des Produktes wird dieses auf Raumtemperatur abgekühlt.

## Patentansprüche

1. Verfahren zum Konservieren von lebenden oder lebensfähigen Mikroorganismen, bei welchem Mikroorganismen mit einem flüssigen Kulturmedium eine Mikroorganismensuspension bilden, mit welchem pulverförmiges poröses Substrat beimpft wird, wobei sich Mikroorganismen in und an Partikeln des porösen Substrates anlagern, und
anschließend die mit den Mikroorganismen versehenen Partikel des Substrates mit einem Hüllmaterial luftdicht umschlossen und zu Formkörpern geformt werden, wobei unter Verwendung eines Extruders zunächst das Hüllmaterial und anschließend das mit den Mikroorganismen beimpfte poröse Substrat in den Extruder eingeleitet und darin zu einer Formmasse vermischt werden, welche zu den Formkörpern ausextrudiert wird.

2. Verfahren nach Anspruch 1,
**dadurch gekennzeichnet,**
**dass** das pulverförmige poröse Substrat Kieselgur umfasst.

3. Verfahren nach Anspruch 1 oder 2,
**dadurch gekennzeichnet,**
**dass** als Hüllmaterial ein thermoplastisches wasserlösliches Polymer, insbesondere Polyethylenglykol (PEG), eingesetzt wird.

4. Verfahren nach einem der Ansprüche 1 bis 3,
**dadurch gekennzeichnet,**
**dass** das flüssige Kulturmedium ein Komplexmedium oder ein definiertes synthetisches Medium ist.

5. Verfahren nach einem der Ansprüche 1 bis 4,
**dadurch gekennzeichnet,**
**dass** zum Bilden der Formmasse eine Trägersubstanz in den Extruder eingeleitet wird.

6. Verfahren nach Anspruch 5,
**dadurch gekennzeichnet,**
**dass** die Trägersubstanz zusammen mit dem Hüllmaterial in den Extruder eingeleitet wird.

7. Verfahren nach Anspruch 5 oder 6,
**dadurch gekennzeichnet,**
**dass** die Trägersubstanz Bentonit, Rapsschrot, Dicalciumphosphat und/oder Kreide umfasst.

8. Verfahren nach einem der Ansprüche 1 bis 7,
**dadurch gekennzeichnet,**
**dass** das Hüllmaterial in einem festen Zustand in den Extruder eingeleitet, darin aufgeschmolzen und bei einer Temperatur von nicht mehr als 55°C mit dem mit den Mikroorganismen beimpften Substrat vermischt wird.

9. Verfahren nach einem der Ansprüche 1 bis 8,
**dadurch gekennzeichnet,**
**dass** die Formmasse am Austritt aus dem Extruder durch eine Lochmatrize zu Strängen geformt wird, welche zu Granulat abgelängt werden.

10. Mikroorganismen-Substanz, welche mit einem pulverförmigen porösen Substrat gebildet ist, deren Partikel mit lebenden oder lebensfähigen Mikroorganismen beimpft sind,
wobei zum Konservieren der Mikroorganismen die mit Mikroorganismen beimpften Partikel des Substrates mit einem Hüllmaterial luftdicht umschlossen und zu Formkörpern ausextrudiert sind, wobei
das poröse Substrat Kieselgur und das Hüllmaterial ein thermoplastisches wasserlösliches Polymer enthält sowie ferner eine Trägersubstanz mit einem Massenanteil zwischen 60% und 90% vorgesehen ist.

11. Substanz nach Anspruch 10,
**dadurch gekennzeichnet,**
**dass** die Trägersubstanz Bentonit, Rapsschrot, Dicalciumphosphat und/oder Kreide mit einem Massenanteil von zumindest 70% umfasst.

12. Substanz nach Anspruch 10 oder 11,
**dadurch gekennzeichnet,**
**dass** die Formkörper als Granulatkörner mit einer Granulatgröße zwischen 1 mm und 15 mm gebildet sind.

## Claims

1. Method for preserving living or viable micro-organisms, in which micro-organisms form a micro-organism suspension with a liquid culture medium, with which a powdery porous substrate is inoculated, wherein micro-organisms adsorb in and on particles of the porous substrate, and
subsequently the particles of the substrate provided with the micro-organisms are sealed hermetically with a coating material and molded to molded bodies, wherein, by making use of an extruder, initially the coating material and subsequently the porous substrate inoculated with the micro-organisms are introduced into the extruder and mixed therein to a molding compound which is extruded into the molded bodies.

2. Method according to claim 1,
**characterized in that**
the powdery porous substrate comprises kieselguhr.

3. Method according to claim 1 or 2,
**characterized in that**
as coating material a thermoplastic water-soluble polymer, in particular polyethylene glycol (PEG), is used.

4. Method according to any one of claims 1 to 3,
**characterized in that**
the liquid culture medium is a complex medium or a defined synthetic medium.

5. Method according to any one of claims 1 to 4,
**characterized in that**
to form the molding compound a carrier substance is introduced into the extruder.

6. Method according to claim 5,
**characterized in that**
the carrier substance is introduced together with the coating material into the extruder.

7. Method according to claim 5 or 6,
**characterized in that**
the carrier substance comprises bentonite, rapeseed meal, dicalcium phosphate and/or chalk.

8. Method according to any one of claims 1 to 7,
**characterized in that**
the coating material is introduced in a solid state into the extruder, melted therein and mixed at a temperature not exceeding 55 °C with the substrate inoculated with the micro-organisms.

9. Method according to any one of claims 1 to 8,
**characterized in that**
at the exit of the extruder the molding compound is molded by a perforated die into strands which are cut into granules.

10. Micro-organism substance which is formed with a powdery porous substrate, the particles of which are inoculated with living or viable micro-organisms,
wherein for the purpose of preserving the micro-organisms the particles of the substrate inoculated with micro-organisms are sealed hermetically with a coating material and extruded into molded bodies, wherein
the porous substrate contains kieselguhr and the coating material a thermoplastic water-soluble polymer and furthermore a carrier substance with a weight percent ranging between 60 % and 90 % is provided.

11. Substance according to claim 10,
**characterized in that**
the carrier substance comprises bentonite, rapeseed meal, dicalcium phosphate and/or chalk with a weight percent of at least 70%.

12. Substance according to claim 10 or 11,
**characterized in that**
the molded bodies are formed as granules with a granule size ranging between 1 mm and 15 mm.

## Revendications

1. Procédé de conservation de micro-organismes vivants ou capables de vivre, selon lequel des micro-organismes forment avec un milieu de culture liquide une suspension de micro-organismes, avec laquelle un substrat poreux pulvérulent est inoculé, les micro-organismes se fixant alors dans et aux particules du substrat poreux, et,
ensuite, les particules du substrat munies des micro-organismes sont entourées d'un matériau de revêtement de manière étanche à l'air et sont constituées en corps formés, tandis que, une extrudeuse étant utilisée, d'abord le matériau de revêtement puis le substrat poreux inoculé avec les micro-organismes sont chargés dans l'extrudeuse et y sont mélangés pour former une masse formée conduisant par extrusion aux corps formés.

2. Procédé selon la revendication 1,
**caractérisé :**
**en ce que** le substrat poreux pulvérulent comprend de la terre de diatomées.

3. Procédé selon la revendication 1 ou 2,
**caractérisé :**
**en ce que**, comme matériau de revêtement, un polymère thermoplastique soluble dans l'eau, en particulier un polyéthylène glycol (PEG), est utilisé.

4. Procédé selon une des revendications 1 à 3,
**caractérisé :**
**en ce que** le milieu de culture liquide est un milieu complexe ou un milieu synthétique défini.

5. Procédé selon une des revendications 1 à 4,
**caractérisé :**
**en ce que**, pour la formation de la masse formée, une substance de support est chargée dans l'extrudeuse.

6. Procédé selon la revendication 5,
**caractérisé :**
**en ce que** la substance de support est chargée dans l'extrudeuse ensemble avec le matériau de revêtement.

7. Procédé selon la revendication 5 ou 6,
**caractérisé :**
**en ce que** la substance de support comprend de la bentonite, du tourteau de colza, du phosphate dicalcique et/ou de la craie.

8. Procédé selon une des revendications 1 à 7,
**caractérisé :**
**en ce que** le matériau de revêtement est chargé dans l'extrudeuse dans un état solide, puis y est fondu et, à une température ne dépassant pas 55 °C, y est mélangé avec le substrat inoculé avec les micro-organismes.

9. Procédé selon une des revendications 1 à 8,
**caractérisé :**
**en ce que**, à la sortie de l'extrudeuse, la masse formée est, à l'aide d'une matrice à perforation, mise en forme de fibres, qui sont tronçonnées pour donner un granulat.

10. Substance de micro-organismes, qui est constituée d'un substrat poreux pulvérulent, dont des particules sont inoculées avec des micro-organismes vivants ou capables de vivre,
dans laquelle, pour la conservation des micro-organismes, les particules du substrat inoculées avec les micro-organismes sont entourées d'un matériau de revêtement de manière étanche à l'air et extrudées pour constituer des corps formés,
le substrat poreux contenant de la terre de diatomées et le matériau de revêtement contenant un polymère thermoplastique soluble dans l'eau, et, de plus, une substance de support étant prévue dans une proportion en masse comprise entre 60 % et 90 %.

11. Substance selon la revendication 10,
**caractérisé :**
**en ce que** la substance de support comprend de la bentonite, du tourteau de colza, du phosphate dicalcique et/ou de la craie dans une proportion en masse d'au moins 70 %.

12. Substance selon la revendication 10 ou 11,
**caractérisé :**
**en ce que** les corps formés sont constitués sous la forme de grains de granulat avec une taille des grains comprise entre 1 mm et 15 mm.
